# EUROPEAN PATENT SPECIFICATION

(11) **EP 1 121 954 B1**
(45) Date of publication and mention of the grant of the patent: **23.03.2005**
(21) Application number: 01301050.9
(22) Date of filing: 21.11.1997
(51) Int. Cl.: A61M 25/00

(54) **Multiple lumen access device**
Mehrlumige Zugangsvorrichtung
Dispositif d'accès à plusieurs lumières

(30) Priority: 26.11.1996 US 756763; 17.10.1997 US 953105
(43) Date of publication of application: 08.08.2001
(62) Divisional of application: 97949506.6
(73) Proprietor: Edwards Lifesciences Corporation, Irvine, CA 92614 (US)
(72) Inventor: Mooney, Charles, Costa Mesa, CA 92626 (US); Pecor, Robert, Aliso Viejo, CA 92656 (US); Bobo, Donald, E., Jr, Santa Ana, CA 92706 (US); Higgins, Michael, J., Trabuco Canyon, CA 92679 (US)
(74) Representative: Baker, Colin John

(56) References cited:
- EP-A- 0 381 062
- EP-A- 0 495 263
- EP-A- 0 593 181
- US-A- 5 464 398

## Description

### BACKGROUND OF THE INVENTION

### Field of the Invention

The present invention relates generally to medical devices which are used to provide access into the human body. More particularly, the present invention is directed to access devices which are used to provide a single, relatively long-term, entry port into the body. The entry port is used by doctors and other medical professionals to selectively introduce a variety of medical implements into the body during *in vivo* diagnostic testing and/or treatment protocols.

### Description of Related Art

A wide variety of medical devices have been developed in recent years for providing access into the human blood stream. These devices have traditionally been divided into two different groups based on their function and purpose. The first group of devices includes catheters which are designed to introduce therapeutic and/or diagnostic fluids into the blood stream. The second group includes devices commonly referred to as "introducers" which are designed to provide an access port into the body through which various medical implements may be passed for therapeutic and/or diagnostic purposes.

Central venous catheters are relatively long tubular devices which have tapered distal tips which are designed for entry into central veins to provide a dedicated route of fluid infusion into the body. The original venous catheters were single lumen devices which provided the ability to infuse a single liquid into the vein at one time. Multiple lumen catheters have since been developed which allow simultaneous introduction of two or more liquids into the vein. The portion of the catheter which remains outside of the body has been continually refined and redesigned to provide a low profile which increases comfort and reduces the awkwardness associated with a dedicated tube exiting the body. The central venous pressure catheter is a type of common multiple lumen catheter which allows the simultaneous introduction and withdrawal of fluids as well as the capability of monitoring blood pressure and other vital parameters.

Introducers are substantially different from catheters in both design and purpose. An introducer is an access device which is intended to provide a dedicated access port into the body. Catheters, on the other hand, are intended to be used to infuse or exfuse fluids from the body. Introducers typically include a relatively short lumen through which various medical implements, including catheters, can be selectively introduced and removed from the body. An important feature of any introducer is the valve assembly. The valve assembly provides a constant seal between the blood stream and the *in vitro* environment as medical implements are introduced and withdrawn from the body. The valve assembly is typically located outside of the body at the proximal end of the introducer. As a result, the proximal end of introducers have tended to be relatively bulky.

In addition to a valve assembly, many introducers include a side arm at the proximal end. The side arm is connected to the lumen so that fluids can be introduced into the body simultaneously with the medical device. The introducer lumen is considered to be a "shared" lumen in that the lumen provides a common conduit for both medical implements and fluid pharmaceuticals or diagnostics.

The currently available introducers and other access devices are well-suited for their intended purpose. However, new medical treatments and diagnostic procedures are continually being developed which require more versatile access into the body. For example, organ transplant procedures and cardiac angioplasty require the introduction of complex combinations of medical implements and diagnostic/therapeutic fluids into the body. Many of the presently available access devices are not well-suited for these relatively complex procedures. As a result, multiple access devices are required which must be located at multiple access sites. Accordingly, there is a continuing need to provide improved access devices that have additional capabilities which increase their versatility and usefulness for the increasing variety of invasive treatments and procedures.

EP 0 495 263 assigned to the Kendall Company, discloses a multi-lumen catheter which may be quickly and easily inserted into the body without the use of a dilator. The necessity of using a dilator adds complexity to the process.

EP 0 593 181 in the name of Cook Incorporated, discloses a dual-lumen introducer sheath wherein the second lumen is of smaller size than the first lumen. This invention can provide passage for wire guides, catheters and other devices in addition to providing a method for allowing the injection of fluids into the body.

### SUMMARY OF THE INVENTION

In accordance with the present invention, an improved access device is provided which is designed to provide selective introduction of medical implements into the body while simultaneously providing auxiliary access through dedicated multiple lumens. The present invention is an improvement over existing introducers and other access devices in that multiple lumen access is provided through the introducer in addition to the shared lumen which is used for both medical implements and fluid pharmaceuticals or diagnostics. As an advantage, the improved access device reduces the number of devices required to introduce multiple implements and fluids into the body during complex surgical and diagnostic procedures.

The present invention desirably includes a multiple lumen access system for use in providing an entry port into the human body for selectively introducing medical implements therethrough and for providing simultaneous auxiliary access into the body. The system includes a multiple lumen access device comprising, an outer tube which has a distal end for introduction into the body and a proximal end which remains outside of the body, the outer tube having an exterior surface and an interior surface defining an access passageway with a cross-sectional area. A device lumen through which medical implements may be passed is defined within the interior surface, the device lumen having a distal end and a proximal end. At least one auxiliary lumen is defined within the interior surface and separately from the device lumen, the auxiliary lumen having a distal end and a proximal end. Finally, a device lumen valve adjacent the proximal end of the device lumen to provide sealing of the device lumen when medical implements are both present and absent from the device lumen.

The multiple lumen access system according to the present invention also includes a junction housing having a proximal end and a distal end to which the proximal end of the outer tube connects. The junction housing includes a main channel in fluid communication with the device lumen and at least one auxiliary channel in fluid communication with the at least one auxiliary lumen, the main channel and auxiliary channel(s) diverging from the outer tube to be non-intersecting in the junction housing.

In one embodiment, the device lumen valve is provided as a part of the junction housing and is in fluid communication with the main channel. A device channel may be formed in the junction housing at an angle with the main channel and terminating at an internal end in fluid communication with the main channel. The device lumen valve is positioned at an external end of the device channel so that medical devices may be inserted therethrough and enter the main channel at an angle. The main channel desirably may continue from the distal end of the junction housing past the device lumen to an opening in the junction housing enabling introduction of fluids therethrough to the main channel. In one embodiment, the device lumen valve is molded separately from the junction housing of a material more rigid than the junction housing and is assembled with the multiple lumen access device by insertion in a cavity formed in the junction housing.

In an alternative embodiment, the main channel and auxiliary channel(s) of the junction housing may be oriented substantially coplanar so that the junction housing is substantially flat, the system further including an extension tube extending from the proximal end of the junction housing and in fluid communication with the main channel wherein the device lumen valve is connected to the extension tube to therefore be in fluid communication with the main channel. A side port in the device lumen valve may be provided enabling infusion of fluids to the extension tube and main channel. Furthermore, mating threaded connectors may be included between the device lumen valve and the extension tube enabling easy removal of the device lumen valve. Finally, a luer connector may be provided on the device lumen valve, the system including an infusion syringe having a mating luer connector.

In another embodiment, the present invention is directed to multiple lumen access devices which may include an outer tube which has a distal end for introduction into the body and a proximal end which remains outside of the body. The outer tube may have an exterior surface and an interior surface, the interior surface defining an access passageway which has a cross-sectional area which may vary at different locations between the distal and proximal ends of the outer tube. A device lumen is located within the access passageway and also includes a distal end and proximal end. The device lumen is formed by an inner tube which has an exterior surface and an interior surface. The interior surface defines the device lumen through which medical implements may be passed. At least one auxiliary lumen is located between the exterior surface of the inner tube and the interior surface of the outer tube. In addition, the multiple lumen access device in accordance with the present invention includes a device lumen valve associated with the proximal end of the device lumen to provide sealing of the device lumen when medical implements are both present and absent.

As a further desirable feature of the present invention, the inner tube is sufficiently flexible to be movable from a relaxed position wherein the device lumen has a first cross-sectional area and expanded or contracted positions wherein the device lumen or passageway has cross-sectional areas which are greater than or less than the first cross-sectional area and less than the cross-sectional area of the access passageway. The flexibility of the inner tube is advantageous in that it allows the insertion of a variety of medical implements having different cross-sectional areas. This flexibility allows, the cross-sectional areas and resultant potential fluid flow rate for the auxiliary lumens and the device lumen to be controlled as desired and maximized.

As another feature of the present invention, two or more auxiliary passageways are provided which are defined by the interior surface of the outer tube, the exterior surface of the inner tube and separation surfaces which provide separation barriers between the auxiliary lumens. The provision of two or more auxiliary passageways allows introduction of additional diagnostics or pharmaceutical liquids simultaneously with introduction of a medical implement through the device lumen. Embodiments of the present invention are also described wherein a single auxiliary lumen is provided.

As an additional feature of the present invention, spacer ribs are provided on the interior surface of the outer tube and/or the interior surface of the inner tube. The spacer ribs are located within the auxiliary lumens to prevent complete closure of the lumens during insertion of relatively large medical implements into the device lumen. The spacer ribs located on the interior surface of the inner tube insure that there is a passageway around devices located within the device lumen.

The above-described and many other features and attendant advantages of the present invention will become better understood by reference to the following detailed description when taken in conjunction with the accompanying drawings.

### BRIEF DESCRIPTION OF THE DRAWINGS

FIG. 1 is a perspective view of an exemplary preferred multiple lumen access device in accordance with the present invention.
FIG. 2 is a sectional view of FIG. 1 taken in the 2-2 plane of FIG. 1.
FIG. 3A is a sectional view taken in the same 2-2 plane of FIG. 1 which shows a relatively small diameter medical device located within the device lumen.
FIG. 3B is a sectional view taken in the same 2-2 plane of FIG. 1 showing a relatively large diameter medical implement located within the device lumen.
FIG. 4 is a sectional view of FIG. 1 taken in the 4-4 plane.
FIG. 5 is a sectional view of FIG. 1 taken in the 5-5 plane.
FIG. 6 is a perspective view of a preferred exemplary embodiment in accordance with the present invention.
FIG. 7 is a sectional view of FIG. 6 taken in the 7-7 plane.
FIG. 8 is a sectional view of FIG. 6 taken in the 8-8 plane.
FIG. 9 is a sectional view of a preferred exemplary flexible inner wall showing the location of spacing ribs.
FIG. 10 is a sectional view of a preferred exemplary multiple lumen access device having a single auxiliary lumen.
FIG. 11 is a sectional view of an exemplary multiple lumen access device showing the inner wall in a relaxed condition with a relatively small diameter medical implement located therein.
FIG. 12 is a sectional view of the same multiple lumen access device and medical implement showing the inner wall partially collapsed due to pressurization of the auxiliary lumens.
FIG. 13 is a sectional view of the same multiple lumen access device and medical implement as FIG. 11 showing the inner tube in a more completely collapsed condition than FIG. 12.
FIG. 14 is a perspective view of a further embodiment of a multiple lumen access device in accordance with the present invention.
FIG. 15 is a sectional view of FIG. 14 taken in the 15-15 plane of FIG. 14.
FIG. 16 is an enlarged perspective view of a junction housing of the device shown in FIG. 14.
FIG. 17 is an enlarged perspective of the junction housing of FIG. 16 with a portion cut away on the longitudinal axis.
FIG. 18 is a perspective assembled view of a valve insert used in the junction housing of FIG. 16.
FIG. 19 is an exploded perspective view of the valve insert of FIG. 18.
FIG. 20 is an elevational view of a multiple lumen access device in accordance with the present invention.
FIG. 21 is a plan view of the multiple lumen access device of FIG. 20 showing more details of an associated catheter system.
FIG. 22 is a perspective view of a proximal end of a low-profile junction housing of the device of FIG. 20.
FIG. 23 is a plan view of an alternative multiple lumen access device with a low profile junction housing.
FIG. 24 is a detailed view of an alternative introducer valve assembly for use in the device of FIGS. 21 or 23.
FIG. 25 is a plan view of an alternative multiple lumen access device having a multi-lumen infusion catheter interfacing with a single lumen introducer.
FIG. 26 is a sectional view of a junction housing used in the device of FIG. 25.
FIGS. 27a-d are schematic sectional views of sheath/lumen configurations for use in the multi-lumen infusion catheter of FIG. 25.
FIG. 28 is an elevational view of a further embodiment of a multiple lumen sheath for use in the device of FIG. 25.
FIG. 29 is a sectional view of the multiple lumen sheath of FIG. 28.

### DESCRIPTION OF THE PREFERRED EMBODIMENTS

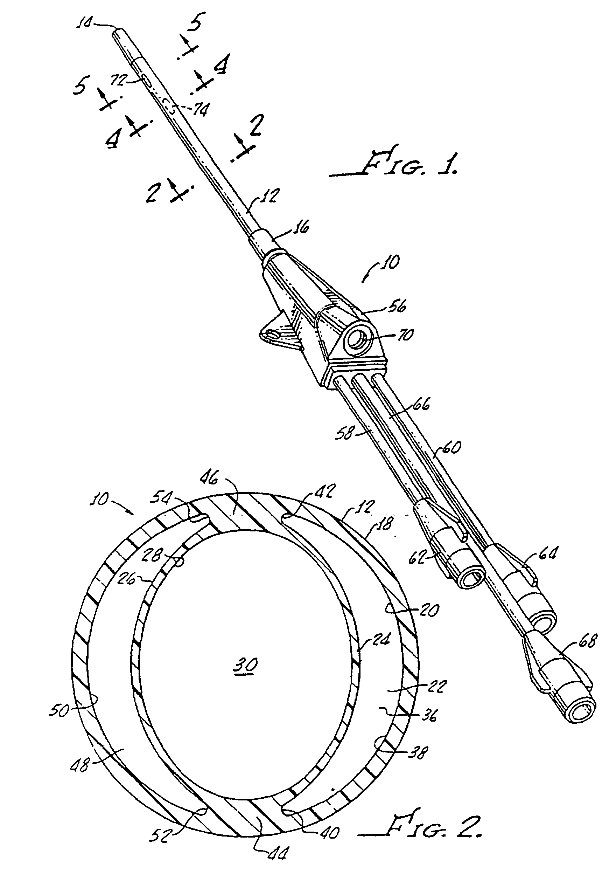

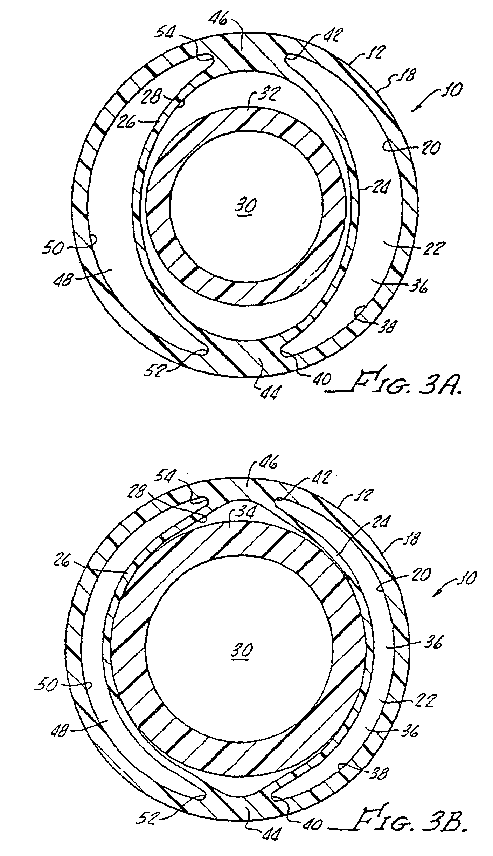

A preferred exemplary multiple lumen access device (MLAD) in accordance with the present invention is shown generally at 10 in FIGS. 1-5. The device 10 includes an outer tube 12 which has a distal end 14 and a proximal end 16. As best shown in FIGS. 2-5, the outer tube 12 has an exterior surface 18 and an interior surface 20. The interior surface 20 defines an access passageway or lumen 22 which has a cross-sectional area that may vary at different locations between the distal 14 and proximal 16 ends of the outer tube 12. Typically, the outer tube 12 may be tapered at the distal end 14, if desired. As a result of the tapering of the outer tube 12, the cross-sectional area will decrease accordingly.

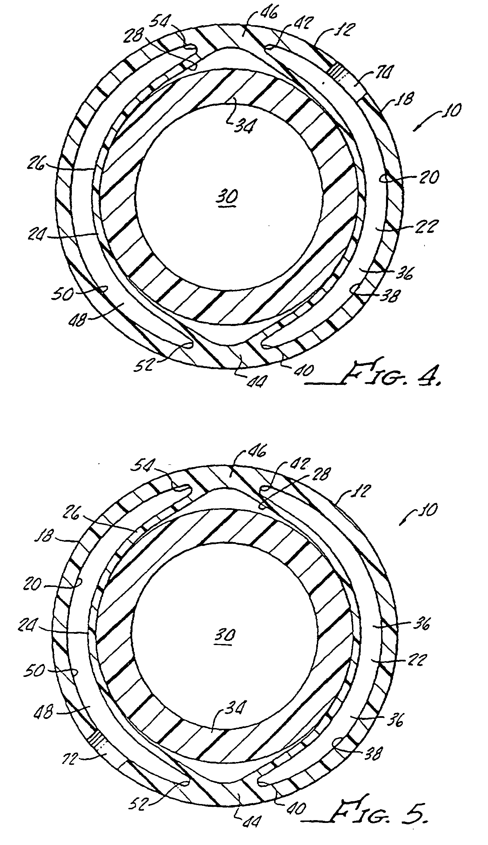

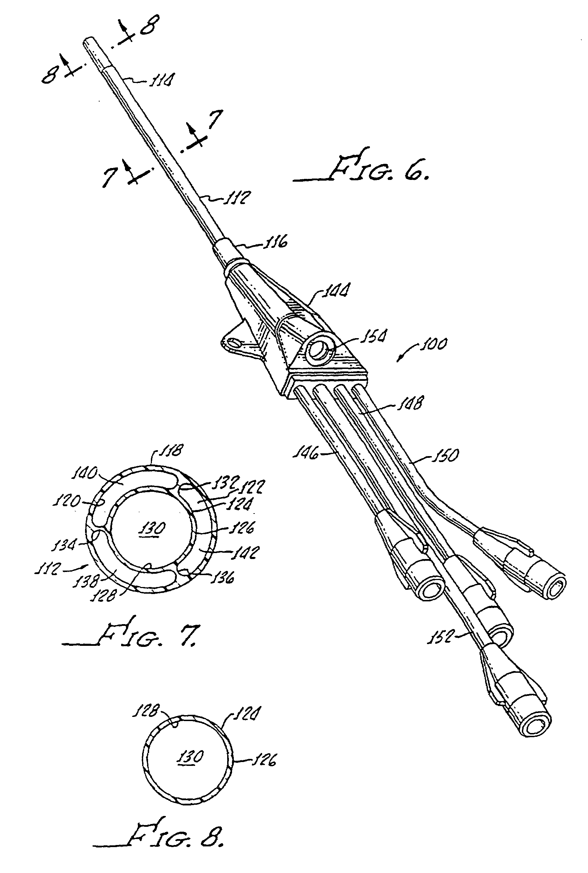

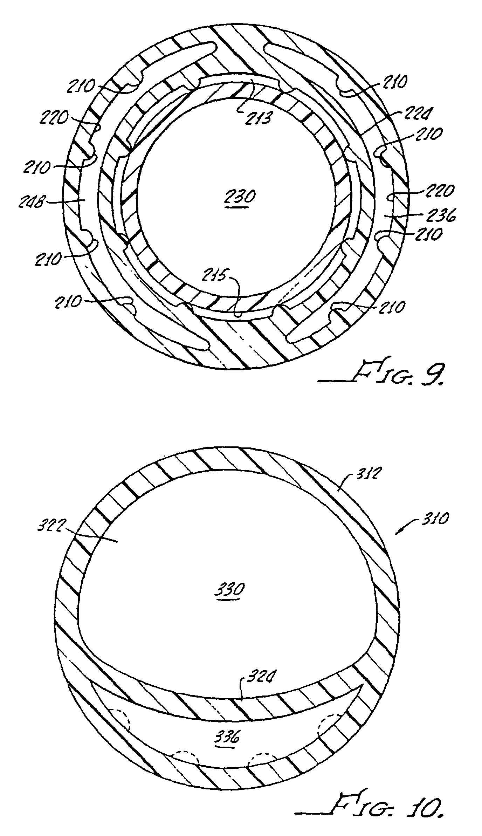

In accordance with the present invention, an inner tube 24 is located within the access passageway 22. The inner tube 24 has a distal end and a proximal end which correspond to the distal end 14 and proximal end 16 of the outer tube 12. The inner tube 24 includes an exterior surface 26 and an interior surface 28. The interior surface 28 defines a device lumen 30 through which medical implements (such as catheters 32 and 34 shown in FIGS. 3A and 3B, respectively) may be inserted into the body. Catheter 34 is also shown in position within the device lumen 30 in FIGS. 4 and 5.

Two auxiliary lumens 36 and 48 are located between the exterior surface 26 of the inner tube 24 and the interior surface 20 of the outer tube 12. The auxiliary lumens 36 and 48 each have a distal end and a proximal end which correspond generally to the distal and proximal ends of the outer tube 12 and inner tube 24. In this particular preferred embodiment, the surfaces which define the auxiliary lumens 36 and 48 correspond to portions of the interior surface of the outer tube and exterior surface of the inner tube. Specifically, auxiliary lumen 36 is defined or bordered by an interior surface 38 which corresponds to the interior surface 20 of the outer tube 12 and the exterior surface 26 of the inner tube 24. Further, the auxiliary lumen 36 is defined by separation surfaces 40 and 42 which are formed by separation barriers 44 and 46, respectively.

A second auxiliary lumen 48 is also formed or defined by the interior surface 20 of the outer tube 12 and the exterior surface 26 of the inner tube 24. Accordingly, the interior surface 50 which defines the second auxiliary lumen 48 corresponds to these surfaces. In addition, the auxiliary lumen 48 is bordered by separation surfaces 52 and 54 formed by separation barriers 44 and 46, respectively.

Referring to FIG. 1, the multiple lumen access device 10 includes a junction housing 56. The junction housing 56 is connected to the proximal end 16 of the access lumen 12. The housing 56 includes infusion tubes 58 and 60 which are connected through the housing 56 to auxiliary lumens 36 and 48, respectively. The infusion tubes 58 and 60 include luer connectors 62 and 64. Other conventional connection devices may be used. A third infusion tube 66 is connected via the housing 56 to the device lumen 30 in order to provide a route for infusion of liquid into the device lumen 30. It should be noted that the infusion tube 66 is not connected to the junction housing 56 at a right angle as is typically done in conventional introducer-type devices. Instead, the infusion tube 66 extends from the housing 56 parallel to the other two infusion tubes 58 and 60. This parallel orientation of the tubes 58, 60 and 66 allows housing 56 to be a low profile body which reduces the bulkiness of the proximal end of the device and increases its wearing comfort. A conventional locking device, such as luer lock 68 is also provided at the proximal end of the infusion tube 66.

The housing 56 includes a valve 70 through which various medical implements are inserted into the device lumen 30. Valve 70 includes a valve or gasket assembly which is designed to provide sealing of the device lumen 30 when medical implements are both present and absent from the device lumen 30. Any of the known gasket arrangements and valve mechanisms used to provide sealing of introducers and related medical implement access devices are suitable. The multiple lumen access device 10 is designed for use in combination with providing access to either the arterial or venous sides of the bloodstream.

An opening 72 (*see* FIG. 1 and FIG. 5) is provided towards the distal end of outer tube 12. The opening 72 is provided to allow exit of fluid from auxiliary lumen 48 which has been introduced through infusion tube 58. Likewise, an opening 74 (shown in phantom in FIG. 1 and also shown in FIG. 4) is provided for allowing the fluid introduced through infusion tube 60 to exit auxiliary lumen 36 at the proximal end of the outer tube 12.

In this exemplary embodiment of the present invention, the inner tube 24 must be sufficiently flexible to be stretchable between a relaxed position as shown in FIG. 3A and various expanded positions as exemplified in FIG. 3B. In FIG. 3A, a catheter 32 having a diameter of 1.3 millimeter (4 french) is shown inserted within the device lumen 30. The inner tube 24 is in a relaxed position where the cross-sectional area of the device lumen 30 is approximately 2 square millimeters. The relaxed cross-sectional area of the device lumen 30 will preferably range from 1 to 3 square millimeters. Larger diameters are possible, if desired. It is preferred, but not required, that inner tube 24 have a circular or elliptical cross-section.

As shown in FIG. 3B, a larger diameter catheter 34 has been inserted into the device lumen 30. The inner wall 24 is made from sufficiently resilient material and is sufficiently sized so that it can expand to the diameter shown which is approximately 3 millimeter (9 french). The maximum diameters to which the inner tube 24 can be expanded is limited by the diameter of the outer tube 12. The inner tube 24 may be flexed inward, if desired, by applying fluid pressure through one or both auxiliary lumens 36 and 48. Typically, the cross-sectional area of the device lumen 30 when the inner tube 24 is in its maximum expanded state will range from 5 to 9 square millimeters. Larger diameters are possible, if desired. Preferably, the inner tube 24 will be sufficiently flexible so that it can be expanded completely outward against the interior surface 20 of the outer tube 12. In the fully expanded state, the auxiliary lumens 36 and 48 will have substantially reduced cross-sectional areas. However, it is preferred that the auxiliary lumens 36 and 48 not be entirely closed. It is desirable to leave some opening through these two auxiliary lumens 36 and 48 at all times to allow flushing fluids to be passed through the lumens in order to prevent the formation of blood clots or other problems associated with a completely collapsed lumen.

Preferably, the inner tube 24 is sufficiently flexible to be stretched to expanded positions wherein the cross-sectional area of the device lumen 30 in the expanded state is up to 85 percent of the cross-sectional area of the access lumen 22. This allows for continual auxiliary fluid introduction through auxiliary lumens 36 and 48. Further, it is preferred that in the relaxed position as shown in FIG. 3, that the device lumen 30 have a cross-sectional area which is not less than 35 percent of the cross-sectional area of the access lumen 22.

In accordance with the present invention, the inner tube 24 is preferably connected to the outer tube 12 at separation barriers 44 and 46 in order to divide the access lumen 22 into a three-chamber lumen, i.e. the central device lumen 30 and two auxiliary lumens 36 and 48. In order to achieve the desired flexibility of the device lumen 30, it is preferred that a relatively elastic material be utilized. Suitable elastic materials include, but are not limited to, polyvinylchloride, polyurethane, polyethylene, nylon, silicone, fluoropolymers and polypropylene. Further, in order to achieve the desired variation in lumen cross-sectional areas, the thickness and durometer of the inner tube walls 24 must be carefully matched to the particular material being utilized. For less flexible materials, the wall thicknesses must be correspondingly reduced in order to achieve the desired flexibility limits. The inner tube 24 should be sufficiently flexible so that it can be expanded to diameters which are at least as large as the outer tube 12.

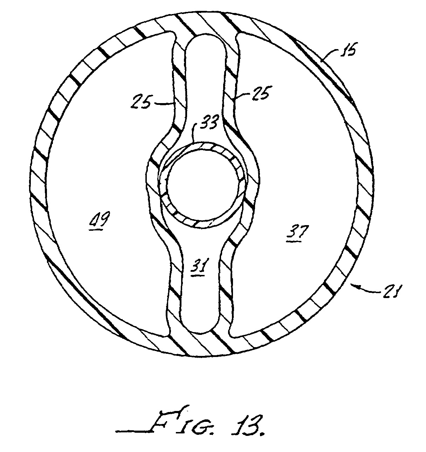

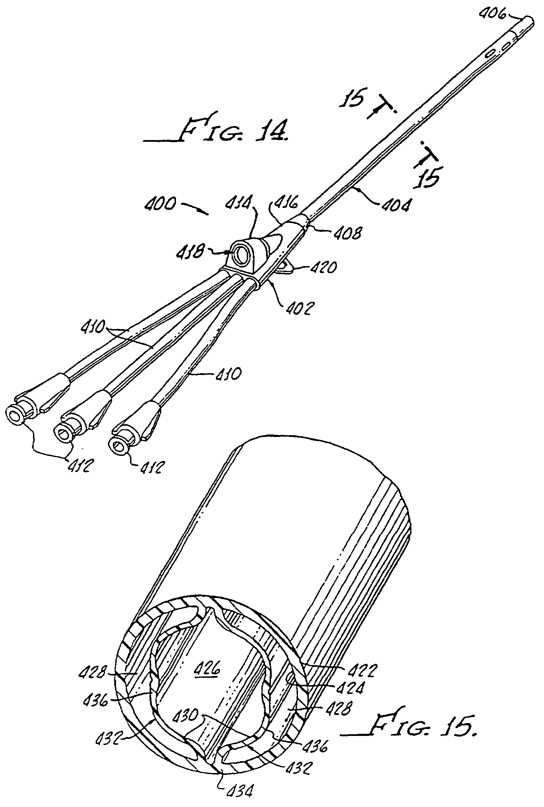

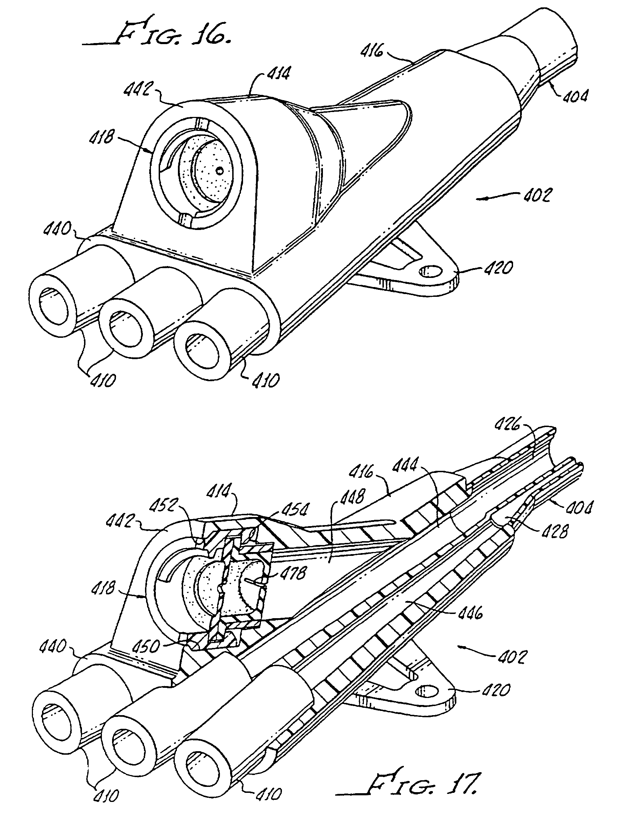

In a preferred embodiment, as shown in FIG. 9, spacer ribs 210 are provided on the interior surface 220 of the outer tube 212 to prevent the inner tube 224 from being expanded to a position which closes the auxiliary lumens 236 and 248. Spacer ribs 211 may also be provided to insure that a passageway 213 is maintained around a device 215 when it is located within device lumen 230. The ribs 210 are preferably located longitudinally along the entire length of the outer tube 212 where the inner tube 224 is also present. The particular cross-sectional shape of the spacer ribs 210 is not particularly important so long as they are relatively blunt and do not damage the inner tube 224 during contact therewith. The number and relative positioning of the spacer must be chosen to insure that complete closure of the auxiliary lumens 236 and 248 does not occur. For inner tubes 224 which are relatively flexible, the number and size of ribs may have to be increased. The ribs 210 shown in FIG. 9 are an example of a preferred configuration. The number, shape, size and position of the ribs 210 may be varied as required in order to prevent closure of the auxiliary lumens 236 and 248 as discussed above.

Although more than two auxiliary lumens may be included into the access device, it is preferred that two lumens be utilized. The use of two lumens is a preferred design for allowing uniform expansion of the inner tube 24 between the relaxed state as shown in FIG. 3A and an expanded state as shown in FIG. 38.

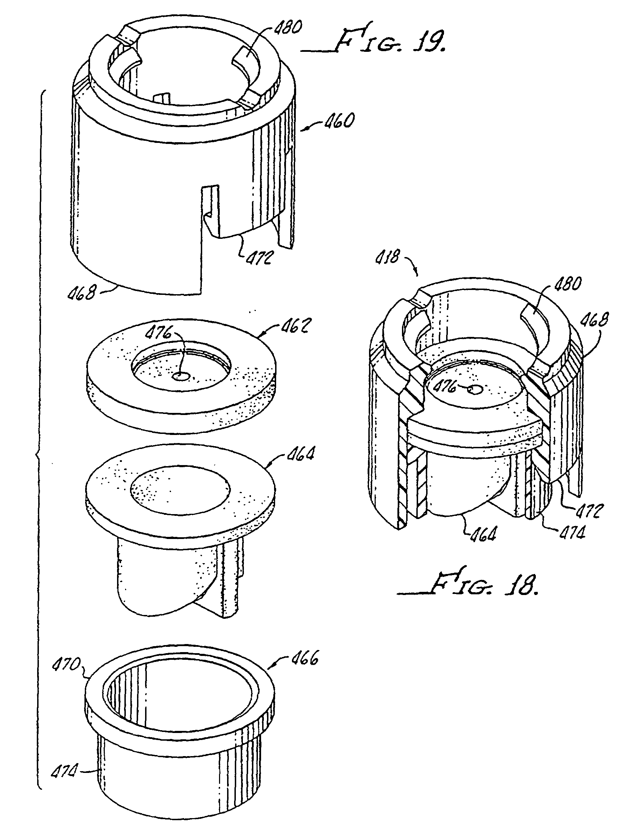

Access devices which include one auxiliary lumen are also possible. The cross-section of an exemplary access lumen is shown at 310 in FIG. 10. The access lumen 310 includes an outer tube 312 which defined an access lumen 322. The access lumen 322 is divided into a device lumen 330 and an auxiliary lumen 336 by an inner flexible wall 324. The inner surface of the outer wall 312 preferably includes spacer ribs (shown in phantom at 350) to prevent closure of the auxiliary lumen 336. The inner wall 324 is made from the same types of flexible materials as described previously for the inner tubes used in the multiple auxiliary lumen embodiments. This particular embodiment is well-suited for use in those situations where a relatively large device lumen is required in favor of the advantages provided by multiple auxiliary lumens.

The outer wall 12 is preferably made from any of the well-known polymer materials used in fabricating introducers and other access devices. Exemplary materials include polyurethane, polyethylene, polypropylene, nylon, polyester, polyether/ester copolymers, silicone based polymers, metalocene catalyzed polyolefins or ethylene vinyl acetate and synthetic rubbers. Preferably, the material used and wall thicknesses for the outer wall 12 are such that the outer wall 12 is a relatively stiff tube in relation to the inner tube 24. Further, the material used for the outer wall 12 should be compatible for molding purposes with the material used to form the inner wall 24. It is preferred that the outer wall 12 and inner wall 24 be coextruded and that the separation barriers 44 and 46 be formed by molding of the two tubes together during the extrusion process. The outer wall 12 and inner wall 26 may be made from the same material or different materials. The inner wall 26 is preferably made from softer versions of the various polymers listed above. When using different materials, the materials must be compatible for bonding or fusing together.

Other fabrication techniques for connecting the inner and outer tubes are possible provided that the connection between the two lumens at the separation barriers 44 and 46 extends the entire length of the two lumens and provides a solid integral connection between the lumens. For example, radio frequency (RF) welding of the tubes is another possible fabrication procedure which may be used to make the access lumen in accordance with the present invention. If desired, the entire triple lumen can be extruded as a single integral multiple lumen structure.

During use, the exemplary access devices 10 allows introduction of medical implements into the device lumen while at the same time allowing infusion of fluid through lumen 68 also into device lumen as well as infusion through lumens 62 and 64 into auxiliary lumens 48 and 36, respectively. Since, as discussed above, the outer tube 12 is relatively inflexible, the total available cross-sectional area for insertion of medical implements and flow of fluids is limited for a given access device. However, the flexibility of the device lumen allows the doctor or other medical professional to selectively and fully utilize the total available cross-sectional area.

In FIG. 3A, a relatively small catheter 32 is shown inserted within the device lumen 30. In this configuration, fluids may be infused/removed through the unused area of the device lumen 30 as well as the two auxiliary lumens 36 and 48. It should be noted that the preferred design inherently centers the catheter or medical implement 32 so that the auxiliary lumens 36 and 48 have approximately equal cross-sectional areas. However, it should be noted that the application of differential pressure to the infusion lumens 58 and 60 can be used to selectively increase or decrease the relative cross sectional areas available for infusion of fluids through the auxiliary lumens. For example, the size of auxiliary lumen 36 can be increased relative to the cross-sectional size of auxiliary lumen 48 by introducing the infusion of liquid through lumen 58 at a pressure which is relatively higher than that of lumen 60. The double auxiliary lumen design of this preferred exemplary embodiment is especially well suited for providing such differential fluid flows when desired.

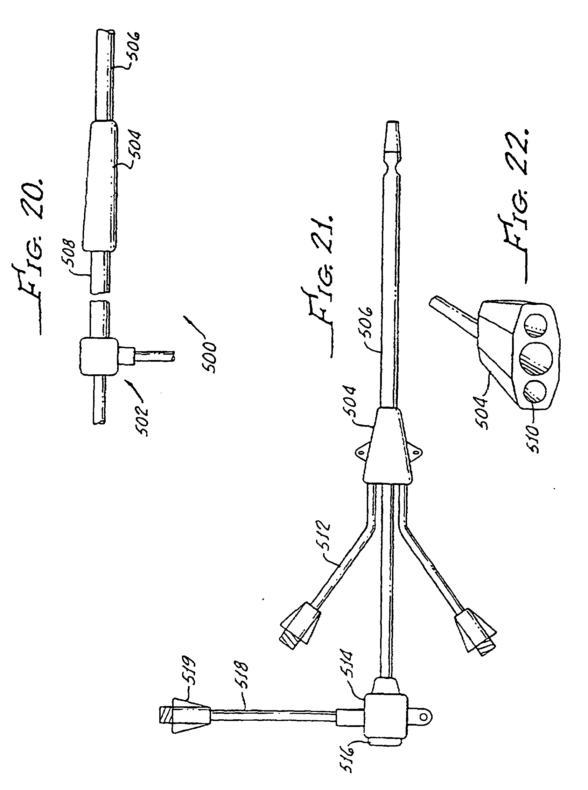

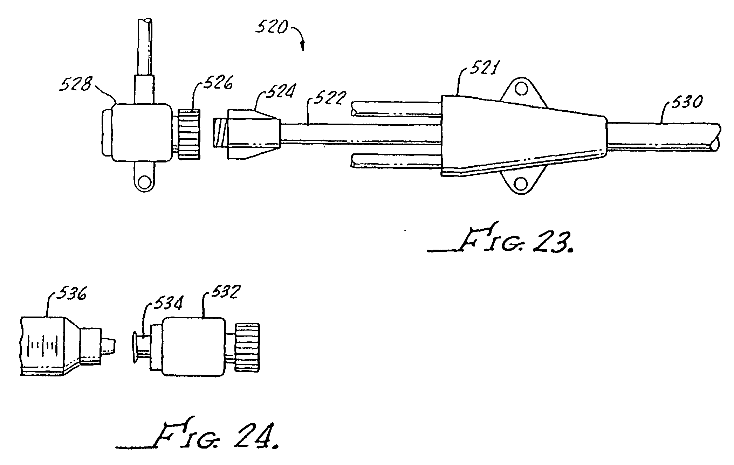

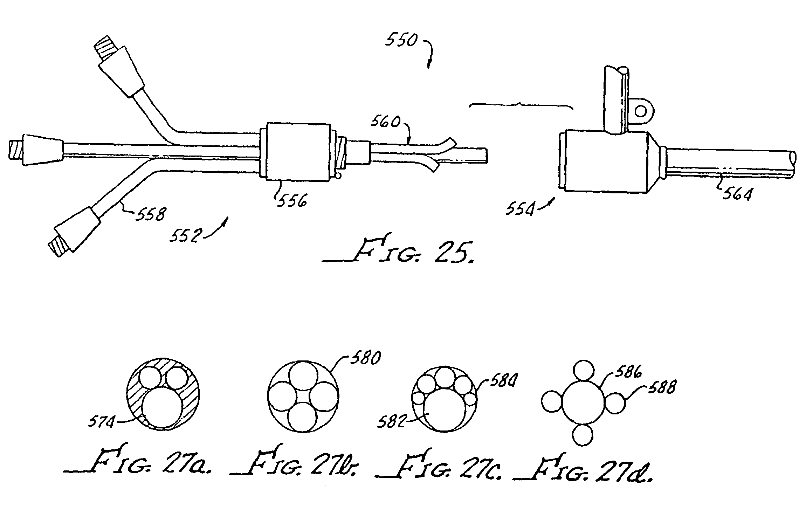

An exemplary embodiment which further demonstrates the flexibility of devices in accordance with the present invention is demonstrated in FIGS. 11-13. In FIG. 11, an exemplary access device 21 is shown in which a relatively small catheter 33 is located within the device lumen 31. In this configuration, fluids may be infused/removed through the unused area of device lumen 31 as well as the two auxiliary lumens 37 and 49. As shown in FIG. 11, the inner flexible wall 25 is in a relaxed position. In this position, the inner wall 25 is relatively close to the outer wall 15. When desired, the size of the auxiliary lumens 37 and 49 can be increased substantially by increasing the pressure of liquids being passed therethrough. The result, as shown in FIG. 12, is the partial collapsing of the inner tube or inner walls 25 about the catheter 33. In the partially contracted or collapsed position as shown in FIG. 12, the inner walls 24 are not stretched. Instead, their configuration changes as shown in FIG. 12 to accommodate the change in relative sizes of the auxiliary lumens and device lumen. As shown in FIG. 13, the size of auxiliary lumens 37 and 49 are increased even further to a point where the fluid flow through the two auxiliary lumens is maximized. In this condition, stretching of the contracted flexible walls 25 may occur. As is apparent from FIGS. 11-13, it is possible to provide a wide variance in fluid flows through the auxiliary lumens and device lumen depending upon differential pressures applied through the various lumens.

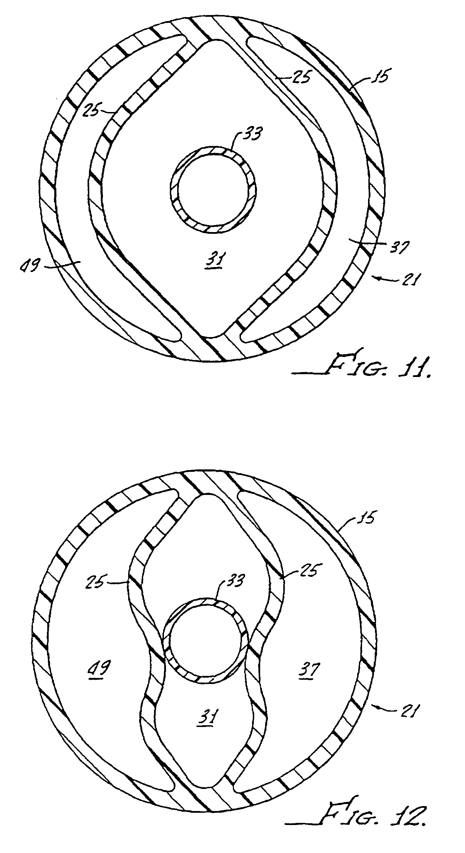

Another preferred exemplary embodiment in accordance with the present invention is shown generally at 100 in FIG. 6. The access device 100 is similar to the previous preferred embodiments in that it includes an outer tube 112 having a distal end 114 and a proximal end 116. As best shown in FIGS. 7 and 8, the outer tube 112 has an exterior surface 118 and an interior surface 120. The interior surface defines an access passageway 122 in which an inner tube 124 is located. The inner tube 124 includes an exterior surface 126 and an interior surface 128. The interior surface 128 of the inner tube 124 defines a device lumen 130 through which medical implements, such as a catheter, may be inserted. The access device 100 includes three separation barriers 132, 134 and 136 which, in combination with the interior surface of the outer tube 120 and exterior surface of the inner tube 126, form three auxiliary lumens 138, 140 and 142. The multiple lumen access device 100 includes the same type of junction housing 144 which was described in the previously-described preferred embodiment (FIGS. 1-5), except that an additional infusion lumen is included to provide infusion of liquid into the additional auxiliary lumen. As shown in FIG. 6, infusion lumens 146, 148 and 150 are connected via junction housing 144 to auxiliary lumens 138, 140 and 142, respectively. A primary infusion lumen 152 is also provided for introducing fluids into the device lumen 130. Again, an access port 154 is provided with the appropriate gaskets and/or valving mechanism to allow introduction of catheters and other medical devices into the device lumen 130.

The inner tube 124 in this preferred exemplary embodiment may or may not be made from flexible material. The inclusion of three separation barriers in this particular embodiment reduces the ability for flexible expansion and contraction of the inner tube 124. However, it is preferred that the material used to form the device lumen 124 and the separation barriers be more flexible than the exterior outer tube 112 in order to allow variations in the cross-sectional areas of the auxiliary lumens. Otherwise, the same materials and fabrication techniques which are used to fabricate the prior embodiments are also suitable for use in making the multiple lumen access device 100.

The above described exemplary embodiments may be used in the same manner as conventional introducer devices. Additionally, if desired, the devices may be used in the same manner as conventional central venous pressure catheters. As will be appreciated by those skilled in the art, the present invention provides the design flexibility to allow use as a single device where the capabilities of an introducer device and catheter are simultaneously required. For example, many diagnostic and invasive medical procedures require the insertion of guide wires and/or medical devices, while simultaneously monitoring critical bodily functions and introducing or removing fluids as needed. The access device of the present invention allows all of the above functions to be performed simultaneously and selectively through a single access device.

### MLAD WITH VALVE INSERT

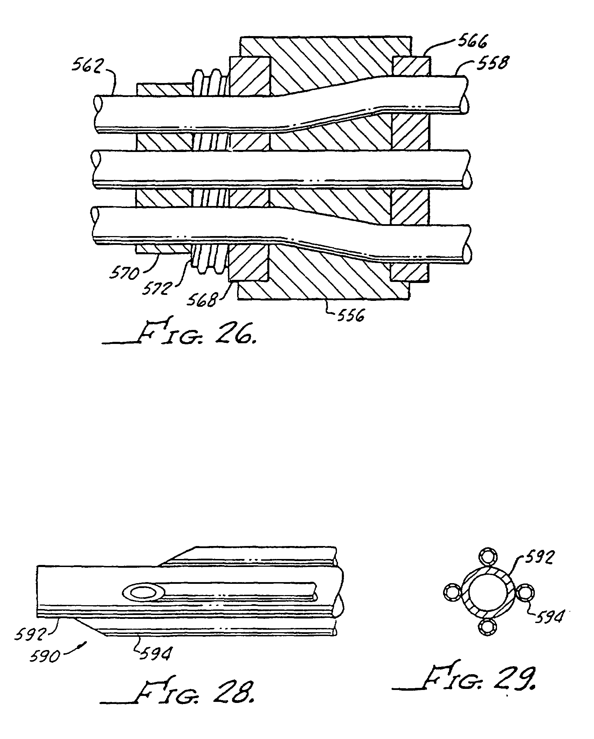

FIG. 14 illustrates an alternative multiple lumen device 400 (MLAD) in accordance with the present invention with an improved junction housing 402. The device 400 is similar to the FIGS. 1-5, and includes a multiple lumen sheath 404 extending distally from the housing 402. The multiple lumen sheath has a distal end 406 for insertion in a body cavity and a proximal end 408 attached to the housing 402. A plurality of extension tubes 410 are attached to the proximal end of the housing 402 and terminate in luer connectors 412. The housing comprises a valve insert portion 414 and a low profile lumen portion 416. A valve insert 418 is secured in a cavity defined in the portion 414. A pair of mounting wings 420 are integrally formed with the junction housing 402 for attaching to a patient.

The multiple lumen sheath 404 seen in cross-section in FIG. 15 comprises an outer circular tube 422 having an interior surface 424. In the illustrated embodiment, the multiple lumen sheath 404 includes a central device lumen 426 and a pair of auxiliary lumens 428 disposed on opposite sides of the device lumen. The device lumen 426 is defined between interior surfaces 430 of a pair of divider walls 432. The divider walls extend in a non-linear fashion substantially across the entire outer tube 422 and terminate at junctions 434. The junctions 434 are spaced a slight distance from one another so that the sheath 404 does not exhibit the separation barriers, as previously described. As illustrated, the device lumen 426 is generally concentrically positioned within the outer tube 422 and has a nominal diameter of slightly greater than half the outer tube 422. Between exterior surfaces 436 of the divider walls 432 and the interior surfaces 424 of the outer tube 422, the auxiliary lumens 428 are formed. The lumens 428 are substantially crescent shaped and are shown identical in size. Of course, as described previously, various other lumen configurations can be provided in the multiple lumen sheath 404.

The junction housing 402 is illustrated in greater detail in FIGS. 16 and 17. The low profile lumen portion 416 has an oval cross-section tapering gradually wider along its long axis from the multiple lumen sheath 404 to a proximal face 440 to which the extension tubes 410 connect. Preferably, the housing 402 is injection molded over free ends of the sheath 404 and tubes 410. The valve housing portion 414 angles upward from one wide surface of the lumen portion 416 and terminates in a proximal face 442. The device access valve insert 418 fits within an angled cavity formed in the valve housing portion 418. With specific reference to FIG. 17, the lumen portion 416 comprises a main channel 444 and a pair of auxiliary channels 446 on either side. The main channel communicates with a central extension tube 410, while the auxiliary channels 446 communicate with the side extension tubes. A device channel 448 defined within the valve housing portion 414 is in communication with the main channel 444 and angles upwardly therefrom to terminate in a widened cavity 450. The cavity 450 receives the valve insert 418 which is held therein by a circumferential lip 452 on the outermost portion of the cavity 450. The cavity 450 continues inward from the lip 452 towards the device channel 448 and narrows at a step 454. The step 454 provides a stop surface against which the valve insert 418 is pressed. Desirably, the insert 418 and cavity 450 are keyed to facilitate insertion in a particular rotational orientation and prevent further rotation.

Now with reference to FIGS. 18 and 19, the device access valve insert 418 is seen in greater detail. The valve insert 418 comprises four components: an outer frame 460, a wiper 462, a valve 464, and a sleeve 466. The assembled valve insert 418 is seen in FIG. 18. The wiper 462 and valve 464 are juxtaposed within an outer wall 468 of the frame 460, and held therein by the interaction between a flange 470 of the sleeve 466 and a pair of cantilevered latches 472 provided on the frame. The sleeve 466 further includes a support tube 474 projecting downward from the flange 470 and surrounding the valve 464. The wiper 462 includes an aperture 476 through which device catheters may be inserted in a sealed fashion. The valve 464 may be a conventional duck-billed valve having a valve slit 478, as seen in FIG. 17. The combination of the wiper 462 and the valve 464 effectively seals the device channel 448 formed within the junction housing 402 and the exterior of the junction housing when devices are repeatedly introduced and withdrawn through the valve insert 418. The outer wall 468 further includes a pair of partial threads 480 which cooperate with exterior threads on an infusion catheter dilator or contamination shield (not shown).

The entire valve insert 418 is formed separately from the junction housing 402, which is molded from a soft, flexible material, typically a soft thermoplastic material. The softness of the junction housing 402 is important in enhancing patient comfort and flexibility of the entire multi-lumen access device 400 when assembling and mounting to a patient. Conversely, the frame 460 of the valve insert 418 is relatively rigid for supporting the wiper 462 and duck-billed valve 464. The wiper and duck-billed valve are made of elastomeric materials, and the outer wall 468 prevents valve depression or distortion and thus enhances the patency of the seal formed by the valve insert 418. The sleeve 466 stabilizes the elastomeric valve components, and the support tube 474 provides an outer surface against which the duck-billed valve 464 cannot extend past. The rigidity of the valve insert 418 provides structure to facilitate connection of devices thereto. Furthermore, the junction housing 402 is easily injection molded over the sheath 404 and tubes 410 prior to addition of the insert 418, for a simplified manufacturing process.

### MLADS WITH REMOTE INTRODUCER VALVES

FIGS. 20 and 21 illustrate a further embodiment of the multiple lumen access device 500 in which the device access valve 502 is not formed integrally with the junction housing 504. More particularly, as best seen in FIG. 20, the junction housing 504 has a low profile which is slightly greater than the sheath 506 or extension tubes 508 attached thereto. FIG. 22 shows a proximal end of low profile junction housing 504 illustrating three channels 510 formed therein for communication with three extension tubes 512, seen in FIG. 21. A central extension tube 512 connects with a remote introducer valve 514 which has a proximal opening 516 for device catheter access. Within the introducer valve 514, a number of different duck-bill or other valves may be provided to seal the lumen of the extension tube 512 from the exterior. Introducer valve 514 may include a side port extension tube 518 terminating in a luer lumen hub 519 for attaching to infusion fluid sources. Thus, in this alternative configuration, a single needle stick followed by implantation of the multi-lumen sheath 506 is all that is required to obtain the benefits of both an introducer valve and central venous catheter, as described previously.

In a further alternative of the device 500, FIG. 23 illustrates a multiple lumen access device 520 wherein the central extension tube 522 terminates in a luer connector 524. The luer connector 524 is desirably used to mate with a female luer connector 526 of an introducer valve assembly 528. However, in this detachable configuration, various other medical devices having conventional luer fittings may be attached to the luer connector 524 and placed in communication with a central lumen of the multi-lumen sheath 530. FIG. 24 illustrates a further alternative, wherein the introducer valve assembly 532 is provided with a male luer connector 534 on a proximal end to which an infusion syringe 536 may be attached. As can be seen, various configurations are possible with the remote introducer valve assembly 528, and the low profile junction housing 521 is easily molded over the extension tubes and has a reduced size, thus facilitating the manufacturing process.

### MLAD WITH MULTI-LUMEN CATHETER/INTRODUCER COMBINATION

FIG. 25 illustrates a further alternative multiple lumen access device 550 comprising a multi-lumen infusion catheter 552 in combination with a conventional single-lumen introducer valve 554. The multi-lumen infusion catheter 552 includes a junction housing 556 which interfaces a plurality of proximal extension tubes 558 and a multi-lumen sheath 560 extending distally therefrom. FIG. 26 illustrates one way in which the proximal extension tubes 558 can be routed to communicate with a plurality of tubes 562 providing lumens of the multi-lumen sheath 560. The multi-lumen sheath 560 is sized to fit through the introducer valve 554 having a distal sheath 564, and from there into the body. In this manner, a single-lumen introducer may be implanted into the patient and then used further as an access port for the multi-lumen infusion catheter 552. By leaving the introducer in place, only a single stick is necessary to enjoy both introducer and central venous catheter capabilities.

With specific reference to FIG. 26, a proximal insert 566, and a distal insert 568 are mounted around the array of extension tubes 558, and distal tubes 562, respectively. The housing 556 is then formed by injection molding material around and between the inserts 566 and 568. A valve seal expander 570 may be provided to help keep the duck-bill valve within the introducer valve 554 open. Further, locking threads 572 are preferably provided to interface with the introducer valve housing 554.

FIGS. 27A-D show various configurations of the multi-lumen sheath 560. In FIG. 27A, a three-lumen solid configuration having a larger high-pressure lumen 574 is shown. FIG. 27B illustrates a four-lumen embodiment which has an outer sheath 580 so that fluid may be passed between the sheath and the exterior of the four tubes within. FIG. 27C is similar to the four-lumen sheath of FIG. 27B, but includes a single large lumen 582 and a plurality of smaller lumens 584. Finally, FIG. 27D illustrates an arrangement of lumens having a central high-volume high-pressure lumen 586, and a plurality of smaller lumens 588 attached around the circumference in an even array.

FIGS. 28 and 29 illustrate a further embodiment of a multi-lumen sheath 590 having a central, high-pressure tube 592 and a plurality of outer or auxiliary tubes 594.

Accordingly, the present invention is not limited to the specific embodiments as illustrated herein.

## Claims

1. A multiple lumen access system for use in providing an entry port into the human body for selectively introducing medical devices therethrough and for providing simultaneous auxiliary access into the body, the system including a multiple lumen access device (10) comprising :
an outer tube (12) which has a distal end (14) for introduction into the body and a proximale end (16) which remains outside of the body, the outer tube having an exterior surface (18) and an interior surface (20) defining an access passageway (22) with a cross-sectional area;
a device lumen (30) through which medical devices may be passed defined within the interior surface, the device lumen having a distal end and a proximal end;
at least one auxiliary lumen (36,48) defined within the interior surface and separately from the device lumen, the auxiliary lumen having a distal end and a proximal end; and a junction housing (56) having a proximal end and a distal end to which the proximal end of the outer tube connects, the junction housing including a main channel (444) in fluid communication with the device lumen and at least one auxiliary channel (446) in fluid communication with the at least one auxiliary lumen, the main channel and auxiliary channel diverging from the outer tube to be non-intersecting in the junction housing **characterised in that** it comprises :
a device lumen valve (70) adjacent the proximal end of the device lumen to provide sealing of the device lumen when medical devices are both present and absent from the device lumen; and **in that** said housing is made of a soft, flexible material.

2. A multiple lumen access system according to claim 1 wherein the device lumen valve (70) is provided as a part of the junction housing (56) and is in fluid communication with the main channel (444).

3. A multiple lumen access system according to claim 2 further including a device channel (448) in the junction housing (56) formed at an angle with the main channel (444) and terminating at an internal end in fluid communication with the main channel, the device lumen valve (70) being positioned at an external end of the device channel so that medical devices may be inserted therethrough and enter the main channel at an angle.

4. A multiple lumen access system according to claim 3 wherein the main channel (444) continues from the distal end of the junction housing (56) past the device channel (448) to an opening in the junction housing enabling introduction of fluids therethrough to the main channel.

5. multiple lumen access system according to claim 2 wherein the device lumen valve (70) is molded separately from the junction housing (56) of a material more rigid than the junction housing and is retained in a cavity (450) formed in the junction housing.

6. A multiple lumen access system according to claim 5 further including a device channel (448) in the junction housing (56) formed at an angle with the main channel (444) and terminating at an internal end in fluid communication with the main channel, the device lumen valve (70) being positioned in the cavity (460) at an external end of the device channel so that medical devices may be inserted therethrough and enter the main channel at an angle.

7. A multiple lumen access system according to claim 1 wherein the device lumen valve (70) is detachable from the rest of the system.

8. A multiple lumen access system according to claim 5, wherein the device lumen valve (70) includes a rigid outer frame (460) and an inner elastomeric valve member.

9. A multiple lumen access system according to claim 8, wherein the rigid outer frame (460) includes an outer wall defining internal threads for cooperating with external threads on a device coupling.

10. A multiple lumen access system according to any of claims 5-9, wherein the cavity (460) the junction housing has on its outermost portion a circumferential lip (462) for retaining the device lumen valve (70) and the cavity continues inward from the lip and narrows at a step which provides a stop surface limiting insertion of the valve.

11. A multiple lumen access system according to claim 1 wherein the main channel (444) and auxiliary channel(s) (446) are oriented substantially coplanar so that the junction housings (56) is substantially flat, and further including an extension tube (410) extending from the proximal end of the junction housing and in fluid communication with the main channel wherein the device lumen valve (70) is connected to the extension tube to therefore be in fluid communication with the main channel.

12. A multiple lumen access system according to claim 11 further including a side port in the device lumen valve (70) enabling infusion of fluids to the extension tube (410) and main channel (444).

13. A multiple lumen access system according to claim 11 further including mating threaded connectors between the device lumen valve (70) and the extension tube (410) enabling easy removal of the device lumen valve.

14. A multiple lumen access system according to claim 13 further including a luer connector (62, 64) on the device lumen valve (70) and an infusion syringe having a mating luer connector.

15. A multiple lumen access system according to any of clams 1-14 further including at least one flexible wall (24,25) located within the access passageway (22) having a distal end and a proximal end and opposite sides, wherein one side of the wall partly defines the device lumen (30) and wherein the auxiliary lumen (36,45) is located between the other side of the wall and the interior surface (20) of the outer tube (12), the wall being sufficiently flexible to be movable from a relaxed position wherein the device lumen has a first cross-sectional area to flexed positions wherein the device lumen has cross-sectional areas which are greater than or less than the first cross-sectional area and less than the cross-sectional area of the access passageway.

16. A multiple lumen access system according to claim 15 wherein at least two auxiliary lumens (36, 48) are located within the interior surface and at least two auxiliary channels (446) in the junction housing (56).

## Patentansprüche

1. Zugangssystem mit mehreren Lumina zur Verwendung bei der Schaffung einer Zugangsöffnung in den menschlichen Körper, um selektiv medizinische Vorrichtungen durch diese hindurch einzuführen und um gleichzeitig einen Hilfszugang in den Körper zu schaffen, wobei das System eine Zugangsvorrichtung (10) mit mehreren Lumina besitzt und folgendes aufweist:
- ein äußeres Rohr (12), das ein distales Ende (14) zum Einführen in den Körper und ein proximales Ende (16) aufweist, das außerhalb des Körpers verbleibt, wobei das äußere Rohr eine äußere Oberfläche (18) und eine innere Oberfläche (20) aufweist, die eine Zugangspassage (22) mit einer Querschnittsfläche bilden;
- ein Vorrichtungslumen (30), durch welches medizinische Vorrichtungen hindurchgeführt werden können und das innerhalb der inneren Oberfläche gebildet ist, wobei das Vorrichtungslumen ein distales Ende und ein proximales Ende aufweist;
- mindestens ein Hilfslumen (36, 48), das innerhalb der inneren Oberfläche gebildet ist und von dem Vorrichtungslumen getrennt ist, wobei das Hilfslumen ein distales Ende und ein proximales Ende aufweist; und
- ein Anschlußgehäuse (56), das ein proximales Ende und ein distales Ende aufweist, mit welchem das proximale Ende des äußeren Rohres verbunden ist, wobei das Anschlußgehäuse einen Hauptkanal (444) in Fluidverbindung mit dem Vorrichtungslumen und mindestens einen Hilfskanal (446) in Fluidverbindung mit dem mindestens einen Hilfslumen aufweist,
wobei der Hauptkanal und der Hilfskanal von dem äußeren Rohr derart divergieren, daß sie sich in dem Anschlußgehäuse nicht kreuzen,
**dadurch gekennzeichnet,**
**daß** das System ein Vorrichtungslumenventil (70) aufweist, das dem proximalen Ende des Vorrichtungslumens benachbart vorgesehen ist, um eine Abdichtung für das Vorrichtungslumen zu schaffen, sowohl wenn die medizinischen Vorrichtungen in dem Vorrichtungslumen vorhanden sind als auch wenn diese dort nicht vorhanden sind,
und **daß** das Gehäuse aus einem weichen, flexiblen Material besteht.

2. Zugangssystem mit mehreren Lumina nach Anspruch 1,
wobei das Vorrichtutlgslumenventil (70) als Teil des Anschlußgehäuses (56) ausgebildet ist und mit dem Hauptkanal (444) in Fluidverbindung steht.

3. Zugangssystem mit mehreren Lumina nach Anspruch 2,
das ferner einen Vorrichtungskanal (448) in dem. Anschlußgehäuse (56) aufweist, der unter einem Winkel zu dem Hauptkanal (444) ausgebildet ist und an einem inneren Ende in Fluidverbindung mit dem Hauptkanal endet, wobei das Vorrichtungslumenventil (70) an einem äußeren Ende des Vorrichtungskanals derart positioniert ist, daß medizinische Vorrichtungen durch dieses hindurch eingeführt werden können und unter einem Winkel in den Hauptkanal eintreten können.

4. Zugangssystem mit mehreren Lumina nach Anspruch 3,
wobei der Hauptkanal (444) sich von dem distalen Ende des Anschlußgehäuses (56) über den Vorrichtungskanal (448) hinaus bis zu einer Öffnung in dem Anschlußgehäuse fortsetzt, die das Einführen von Fluiden durch ihn hindurch bis zum Hauptkanal ermöglicht.

5. Zugangssystem mit mehreren Lumina nach Anspruch 2,
wobei das Vorrichtungslumenventil (70) getrennt von dem Anschlußgehäuse (56) aus einem steiferen Material als das Anschlußgehäuse geformt ist und in einem Hohlraum (460) gehalten ist, der in dem Anschlußgehäuse gebildet ist.

6. Zugangssystem mit mehreren Lumina nach Anspruch 5,
das ferner einen Vorrichtungskanal (448) in dem Anschlußgehäuse (56) aufweist, der unter einem Winkel zu dem Hauptkanal (444) ausgebildet ist und an einem inneren Ende in Fluidverbindung mit dem Hauptkanal endet, wobei das Vorrichtungslumenventil (70) in dem Hohlraum (460) an einem äußeren Ende des Vorrichtungskanals derart positioniert ist, daß medizinische Vorrichtungen durch dieses hindurch eingeführt werden können und unter einem Winkel in den Hauptkanal eintreten können.

7. Zugangssystem mit mehreren Lumina nach Anspruch 1,
wobei das Vorrichtungslumenventil (70) von dem Rest des Systems abnehmbar ist.

8. Zugangssystem mit mehreren Lumina nach Anspruch 5,
wobei das Vorrichtungslumenventil (70) einen steifen äußeren Rahmen (460) und ein inneres elastomeres Ventilelement aufweist.

9. Zugangssystem mit mehreren Lumina nach Anspruch 8,
wobei der steife äußere Rahmen (460) eine Außenwand aufweist, die ein Innengewinde zum Zusammenwirken mit einem Außengewinde an einer Vorrichtungskupplung bildet.

10. Zugangssystem mit mehreren Lumina nach einem der Ansprüche 5 bis 9,
wobei der Hohlraum (460) in dem Anschlußgehäuse an seinem äußersten Bereich eine Umfangslippe (462) aufweist, um das Vorrichtungslumenventil (70) festzuhalten, und wobei der Hohlraum sich von der Lippe nach innen fortsetzt und an einer Stufe verengt, welche eine Anschlagfläche bildet, die das Einsetzen des Ventils begrenzt.

11. Zugangssystem mit mehreren Lumina nach Anspruch 1,
wobei der Hauptkanal (444) und der Hilfskanal bzw. die Hilfskanäle (446) im wesentlichen koplanar orientiert sind, so daß das Anschlußgehäuse (56) im wesentlichen flach ist, und wobei ferner ein Verlängerungsrohr (410) vorgesehen ist, das sich von dem proximalen Ende des Anschlußgehäuses aus erstreckt und mit dem Hauptkanal in Fluidverbindung steht, wobei das Vorrichtungslumenventil (70) mit dem Verlängerungsrohr verbunden ist, damit es mit dem Hauptkanal in Fluidverbindung steht.

12. Zugangssystem mit mehreren Lumina nach Anspruch 11,
das ferner einen Seitenanschluß in dem Vorrichtungslumenventil (70) aufweist, was eine Infusion von Fluiden zu dem Verlängerungsrohr (410) und dem Hauptkanal (444) ermöglicht.

13. Zugangssystem mit mehreren Lumina nach Anspruch 11,
das ferner mit passenden mit Gewinde versehenen Verbindern zwischen dem Vorrichtungslumenventil (70) und dem Verlängerungsrohr (410) versehen ist, die ein einfaches Entfernen des Vorrichtungslumenventils ermöglichen.

14. Zugangssystem mit mehreren Lumina nach Anspruch 13,
das ferner einen Luer-Verbinder (62, 64) an dem Vorrichtungslumenventil (70) und eine Infusionsspritze mit einem passenden Luer-Verbinder aufweist.

15. Zugangssystem mit mehreren Lumina nach einem der Ansprüche 1 bis 14,
das ferner mindestens eine flexible Wand (24, 25) aufweist, die innerhalb der Zugangspassage (22) angeordnet ist, die ein distales Ende und ein proximales Ende und gegenüberliegende Seiten aufweist,
wobei die eine Seite der Wand teilweise das Vorrichtungslumen (30) bildet und
wobei das Hilfslumen (36, 45) zwischen der anderen Seite der Wand und der Innenoberfläche (20) des äußeren Rohres (12) angeordnet ist,
wobei die Wand ausreichend flexibel ist, um aus einer entspannten Position, in der das Vorrichtungslumen eine erste Querschnittsfläche besitzt, in ausgelenkte Positionen bewegt zu werden, in denen das Vorrichtungslumen Querschnittsflächen aufweist, die größer oder kleiner sind als die erste Querschnittsfläche sowie kleiner als die Quersehnittsfläche der Zugangspassage.

16. Zugangssystem mit mehreren Lumina nach Anspruch 15,
wobei mindestens zwei Hilfslumen (46, 48) innerhalb der inneren Oberfläche angeordnet sind und mindestens zwei Hilfskanäle (446) in dem Anschlußgehäuse (56) vorhanden sind.

## Revendications

1. Système d'accès à lumières multiples utilisable dans la création d'un orifice d'entrée dans le corps humain pour introduire sélectivement des dispositifs médicaux par cet orifice et pour procurer un accès auxiliaire simultané dans le corps, le système incluant un dispositif d'accès à lumières multiples comprenant :
un tube extérieur (12) qui a une extrémité distale (14) pour introduction dans le corps et une extrémité proximale (16) qui reste à l'extérieur du corps, le tube extérieur présentant une surface extérieure (18) et une surface intérieure (20) qui définit un passage d'accès (22) ayant une section transversale ;
une lumière de dispositif (30) dans laquelle on peut faire passer des dispositifs médicaux, définie à l'intérieur de la surface intérieure, la lumière de dispositif ayant une extrémité distale et une extrémité proximale ;
au moins une lumière auxiliaire (36, 48) définie à l'intérieur de la surface intérieure et séparément de la lumière de dispositif, la lumière auxiliaire ayant une extrémité distale et une extrémité proximale ; et un boîtier de jonction (56) ayant une extrémité proximale et une extrémité distale à laquelle se raccorde l'extrémité proximale du tube extérieur, le boîtier de jonction incluant un canal principal (444) en communication de fluide avec la lumière de dispositif et au moins un canal auxiliaire (446) en communication de fluide avec ladite au moins lumière auxiliaire, le canal principal et le canal auxiliaire divergeant à partir du tube extérieur de façon à ne pas être en intersection dans le boîtier de jonction **caractérisé en ce qu'**il comprend un obturateur de lumière de dispositif (70) adjacent à l'extrémité proximale de la lumière de dispositif pour assurer la fermeture étanche de la lumière de dispositif à la fois lorsque des dispositifs médicaux sont présents dans la lumière de dispositif et lorsqu,ils sont absents de celle-ci ; et **en ce que** ledit boîtier est réalisée dans une matière souple et flexible

2. Système d'accès à lumières multiples selon la revendication 1, dans lequel l'obturateur de lumière de dispositif (70) est prévu sous la forme d'une partie du boîtier de jonction (56) et est en communication de fluide avec le canal principal (444).

3. Système d'accès à lumières multiples selon la revendication 2 incluant en outre un canal de dispositif (448) dans le boîtier de jonction (56), incliné par rapport au canal principal (444) et se terminant à une extrémité intérieure en communication de fluide avec le canal principal; l'obturateur de lumière de dispositif (70) étant placé dans la cavité à une extrémité extérieure du canal de dispositif de sorte que des dispositifs médicaux peuvent être insérés à travers ledit obturateur et entrer dans le canal principal suivant un certain angle.

4. Système d'accès à lumières multiples selon la revendication 3, dans lequel le canal principal (444) s'étend à partir de l'extrémité distale du boîtier de jonction (56), devant le canal de dispositif (448), jusqu'à un orifice du boîtier de jonction permettant l'introduction de fluides par cet orifice dans le canal principal.

5. Système d'accès à lumières multiples selon la revendication 2, dans lequel l'obturateur de lumière de dispositif (70) est moulé séparément du boîtier de jonction (56) en une matière plus rigide que le boîtier de jonction et il est retenu dans une cavité (460) formée dans le boîtier de jonction.

6. Système d'accès à lumières multiples selon la revendication 5, incluant en outre un canal de dispositif (448) dans le boîtier de jonction (56), incliné par rapport au canal principal (444) et se terminant à une extrémité intérieure en communication de fluide avec le canal principal, l'obturateur de lumière de dispositif (70) étant placé dans la cavité (460) à une extrémité extérieure du canal de dispositif de sorte que des dispositifs médicaux peuvent être insérés à travers ledit obturateur et entrer dans le canal principal suivant un certain angle.

7. système d'accès à lumières multiples selon la revendication 1, dans lequel l'obturateur de lumière de dispositif (70) est détachable du reste du système.

8. Système d'accès à lumières multiples selon la revendication 5, dans lequel l'obturateur de lumière de dispositif (70) inclut un corps externe rigide (460) et un organe obturateur élastomérique interne.

9. Système d'accès à lumières multiples selon la revendication 5, dans lequel le corps externe rigide (460) inclut une paroi extérieure définissant des filetages intérieurs pour coopérer avec des filetages extérieurs sur un dispositif d'accouplement

10. Système d'accès à lumières multiples selon l'une quelconque des revendications 5 à 9, dans lequel la cavité (450) dans sa partie la plus extérieure une lèvre circonférentielle (452) pour retenir l'obturateur de lumière de dispositif (70) et la cavité s'étend vers l'intérieur à partir de la lèvre et se rétrécie jusqu'à un épaulement qui définit une surface de butée limitant l'insertion de l'obturateur.

11. Système d'accès à lumières multiples selon la revendication 1, dans lequel le canal principal (444) et le ou les canaux auxiliaires (446) sont orientés sensiblement dans un même plan de sorte que le boîtier de jonction (56) est sensiblement plat, et comprenant en outre un tube de prolongement (410) qui s'étend à partir de l'extrémité proximale du boîtier de jonction et est en communication de fluide avec le canal principal, de sorte que l'obturateur de lumière de dispositif (70) est connecté au tube de prolongement pour être ainsi en communication de fluide avec le canal principal.

12. Système d'accès à lumières multiples selon la revendication 11, comprenant en outre un orifice latéral, dans l'obturateur de lumière de dispositif (70), qui permet une injection de fluides vers le tube de prolongement (410) et le canal principal (444).

13. Système d' accés â lumières multiples selon la revendication 11, comprenant en outre des raccords filetés complémentaires entre l'obturateur de lumière de dispositif (70) et le tube de prolongement (410) pour permettre un démontage facile de l'obturateur de lumière de dispositif.

14. Système d'accès à lumières multiples selon la revendication 13, comprenant en outre un raccord luer (62, 64) sur l'obturateur de lumière de dispositif (70) et une seringue d'injection ayant un raccord luer complémentaire.

15. Système d'accès à lumières multiples selon l'une quelconque des revendications 1-14, comprenant en outre au moins une paroi (24, 25) placée dans le passage d'accès (22) ayant une extrémité distale et une extrémité proximale et des côtés opposés, de sorte qu'un côté de la paroi définit en partie la lumière de dispositif (30) et de sorte que la lumière auxiliaire (36, 48) est située entre l'autre côté de la paroi et la surface intérieure (20) du tube extérieur (12), la paroi étant suffisamment flexible pour être déplaçable d'une position de repos, dans laquelle la lumière de dispositif a une première section transversale, à des positions fléchies dans lesquelles la lumière de dispositif a des sections transversales qui sont plus grandes ou plus petites que la première section transversale et plus petites que la section transversale du passage d'accès.

16. Système d'accès à lumières multiples selon la revendication 15, dans lequel au moins deux lumières auxiliaires (35, 48) sont situées à l'intérieur de la surface intérieure et au moins deux canaux auxiliaires (446) dans le boîtier de jonction (56).
